# EUROPEAN PATENT APPLICATION

(11) **EP 4 012 282 A1**
(43) Date of publication of application: **15.06.2022**
(21) Application number: 21209324.9
(22) Date of filing: 19.11.2021
(51) Int. Cl.: F24F 8/22, F24F 8/80, F24F 13/20

(54) **PORTABLE AIR PURIFIER**

(30) Priority: 14.12.2020 KR 20200174528; 30.12.2020 KR 20200188335
(71) Applicant: LG Electronics Inc., SEOUL 07336 (KR)
(72) Inventor: CHOI, Ji Eun, Seoul (KR); PARK, Hyungho, Seoul (KR); HONG, Seongkyeol, Seoul (KR)
(74) Representative: Vossius & Partner Patentanwälte Rechtsanwälte mbB

(57) **Abstract**

The present disclosure relates to a portable air purifier. The portable air purifier includes a housing provided with an entrance part forming a passage for suctioning air, a filter part disposed on an upper side of the entrance part and configured to purify air suctioned into the entrance part and moving upward, an irradiator disposed on a lower side of the filter part, installed at a height where the irradiator overlaps the entrance part and configured to irradiate a light ray for sterilization to the filter part, and a sterilizing and supporting part configured to support the irradiator, fixed to the housing and disposed around an outer perimeter of the irradiator.

## Description

Disclosed herein is a portable air purifier, and more specifically, a portable air purifier that uses a light ray for sterilization to sterilize a filter part.

Air purifiers are devices that are widely used in our daily lives. The devices can filter physical particles such as dust, fine dust, ultra fine dust and the like, chemical substances such as odorant particles, harmful gases and the like, and microorganisms such as germs, viruses and the like, to purify air.

People cannot live without air purifiers in an industrial society since more and more people are greatly affected by fine dust and suffer from allergies. Accordingly, there is a growing demand for the devices. Additionally, as living standards improve, the demand for the air purifiers grows.

Ordinarily, a large-sized air purifier is used in a house that is 100 square meters or greater. The air purifier can be provided with a filter for physical particles such as dust and the like, a filter for chemical substances such as gas and the like, and a filter for microorganisms such as germs, viruses and the like. That is, such a large-sized air purifier capable of accommodating various types of filters can be used in a large space.

However, the large-sized air purifier is rarely used in a small space such as a studio apartment, a space in a vehicle and the like considering space availability, mobility and energy efficiency. Additionally, a user who moves from place to place usually uses a small-sized air purifier. Under the circumstances, there is a growing need for a portable air purifier that is easy to carry.

The portable air purifiers need to be small and lightweight enough for users to carry such that the users easily carry and use the portable air purifiers anywhere. That is, the portable air purifiers are useful for people who often go out and move from place to place instead of staying in a place such as a house.

In a prior art document (KR Patent Publication No. 10-2020-0037187), air is suctioned into a rear surface of a portable air purifier and is discharged from a front surface of the portable air purifier. The air suctioned into the rear surface of the portable air purifier may be discharged from the front surface of the portable air purifier through a filter and an air blowing fan.

A portable air purifier has a limited size such that a user carries the portable air purifier readily. Accordingly, the portable air purifier is not provided with an additional sterilizer for sterilizing the filter inside a housing.

Further, when a light ray for sterilization is used to sterilize the filter, part of the light ray for sterilization can leak out of the housing.

Furthermore, when a light ray for sterilization is used to sterilize the filter, a proper incident angle and an irradiation distance of the light ray for sterilization cannot be properly ensured due to a limited size of the housing. As a result, the filter cannot be sterilized properly.

A background art in relation to the subject matter of the present disclosure is disclosed in KR Patent Publication No. 10-2020-0037187 (titled Portable Air Caring Apparatus and published on April 08, 2020).

The invention is specified by the independent claim. Preferred embodiments are defined in the dependent claims. The present disclosure is directed to a portable air purifier in which a sterilizer for sterilizing a filter part may be installed in a housing.

The present disclosure is directed to a portable air purifier that may block a light ray for sterilization aiming at a filter part from being irradiated out of a housing.

The present disclosure is directed to a portable air purifier in which an incident angle and an irradiation distance of a light ray for sterilization aiming at a filter part may be ensured despite a limited size of a housing.

Aspects according to the present disclosure are not limited to the above ones, and other aspects and advantages that are not mentioned above can be clearly understood from the following description and can be more clearly understood from the embodiments set forth herein. Additionally, the aspects and advantages in the present disclosure can be realized via means and combinations thereof that are described in the appended claims.

In a portable air purifier according to the present disclosure, a sterilizer may be disposed on a lower side of a filter part.

Specifically, the sterilizer configured to irradiate a light ray for sterilization and sterilize the filter part may be disposed on the lower side of the filter part. Additionally, the sterilizer may be disposed on an inner side of the housing, facing an entrance part, and an exterior of the housing may not be scaled up despite the installation of the sterilizer, thereby making it possible to provide a compact-sized portable air purifier and sterilize the filter part.

The portable air purifier according to the present disclosure may block a light ray for sterilization aiming at the filter part from being irradiated out of the housing.

Specifically, a reflector may be disposed around an outer perimeter of an irradiator, and a light ray for sterilization irradiated from the irradiator to the filter part may be blocked from being irradiated out of the entrance part by the reflector.

The portable air purifier according to the disclosure may ensure an incident angle and an irradiation distance of a light ray for sterilization aiming at the filter part despite a limited size of the housing.

Specifically, the reflector disposed around the outer perimeter of the irradiator may have a funnel shape to reflect a light ray for sterilization to the filter part, and may include a reflective coating to increase reflectivity. Thus, a proper incident angle and irradiation distance of a light ray for sterilization may be ensured.

A portable air purifier in one embodiment may include a housing provided with an entrance part forming a passage for suctioning air, a filter part disposed on an upper side of the entrance part and configured to purify air suctioned into the entrance part and moving upward, an irradiator disposed on a lower side of the filter part, installed at a height where the irradiator overlaps the entrance part and configured to irradiate a light ray for sterilization to the filter part, and a sterilizing and supporting part configured to support the irradiator, fixed to the housing and disposed around the outer perimeter of the irradiator.

The irradiator may include a printed circuit board supported by the sterilizing and supporting part and disposed on the lower side of the filter part, and a sterilizing light source disposed on the printed circuit board and configured to irradiate a light ray for sterilization to the filter part.

Additionally, centers of the sterilizing light source and the filter part may be aligned with each other in an up-down direction.

The sterilizing and supporting part may include a support body disposed around the outer perimeter of the irradiator and configured to reflect a light ray for sterilization irradiated from the sterilizing light source to the filter part.

The support body may include a reflector being concave toward the filter part, provided with the sterilizing light source at a center thereof, and configured to reflect light irradiated from the sterilizing light source to the filter part, and an inner supporter extended to a lower side of the reflector and configured to support the printed circuit board of the irradiator.

The support body may further include a heat dissipating hole part forming a plurality of holes on the reflector and configured to air-cool the irradiator.

The heat dissipating hole part may be provided with a plurality of long holes having a circular arc shape around the sterilizing light source.

The reflector may be disposed on an inner side of the housing, facing the entrance part.

The reflector may include a first reflecting body disposed around a perimeter of the sterilizing light source and spaced from the sterilizing light source, and a second reflecting body extended from the first reflecting body upward at a slant.

The second reflecting body may be formed into a funnel an upper side of which is open, and may have an inner diameter that gradually increases from the first reflecting body toward the filter part.

The inner supporter may include an inner bracket extended to the lower side of the reflector, and a held hook bent from a lower side of the inner bracket inward to the irradiator and held at an edge of the irradiator.

The support body may further include a blocking body extended downward from an outer edge of the reflector.

The blocking body may be formed into a cylinder a lower side of which is open, and a center of the blocking body and a center of the entrance part may be aligned with each other

The sterilizing and supporting part may further include a support bracket extended to a lower side of the support body and fixed to the housing.

A portable air purifier in one embodiment may include a first case provided therein with an accommodation space, provided with an entrance part for suctioning air on a lateral surface of a lower portion thereof and provided with an outlet for discharging air on an upper side thereof, a second case connected to the lower portion of the first case, a filter part installed inside the first case and configured to purify air suctioned into the entrance part, a fan module disposed between the filter part and the outlet and configured to rotate a fan to blow air toward the outlet, an irradiator disposed on a lower side of the filter part and configured to irradiate a light ray for sterilization to the filter part, and a sterilizing and supporting part configured to support the irradiator, fixed to at least one of the first case and the second case and disposed around an outer perimeter of the irradiator.

Additionally, centers of the first case, the filter part, the fan module and the irradiator may be aligned with one another in an up-down direction.

The sterilizing and supporting part may include a support body disposed around the outer perimeter of the irradiator and configured to reflect a light ray for sterilization irradiated from the irradiator to the filter part, and a support bracket extended to a lower side of the support body and fixed to at least one of the first case and the second case.

The support body may include a reflector being concave toward the filter part, provided with the irradiator at a center thereof, and configured to reflect light irradiated from the irradiator to the filter part, and an inner supporter extended to a lower side of the reflector and configured to support a printed circuit board of the irradiator.

The support body may further include a heat dissipating hole part forming a plurality of holes on the reflector and configured to air-cool the irradiator.

Further, a reflective coating layer may be formed on a surface of the reflector.

A portable air purifier according to the disclosure may prevent pollution of air since a filter part is sterilized by a light ray for sterilization irradiated from a sterilizer.

The portable air purifier according to the disclosure may ensure space availability and have a compact size since the sterilizer is installed in a space where an entrance part is installed.

The portable air purifier according to the disclosure may prevent a light ray for sterilization aiming at the filter part from being irradiated out of a housing such that the portable air purifier is reliably used.

The portable air purifier according to the disclosure may reliably ensure an incident angle and an irradiation distance of a light ray for sterilization even in a limited area as a result of installation of a reflector provided with a reflective coating such that the sterilizer provides improved sterilization efficiency.

Specific effects are described along with the above-described effects in the section of Detailed Description.

### BRIEF DESCRIPTION OF DRAWING

The accompanying drawings constitute a part of the specification, illustrate one or more embodiments in the disclosure, and together with the specification, explain the disclosure, wherein:
FIG. 1 is a cross-sectional view showing a portable air purifier of a first embodiment;
FIG. 2 is a cross-sectional view separately showing the portable air purifier of the first embodiment;
FIG. 3 is a perspective view separately showing the portable air purifier ofthe first embodiment;

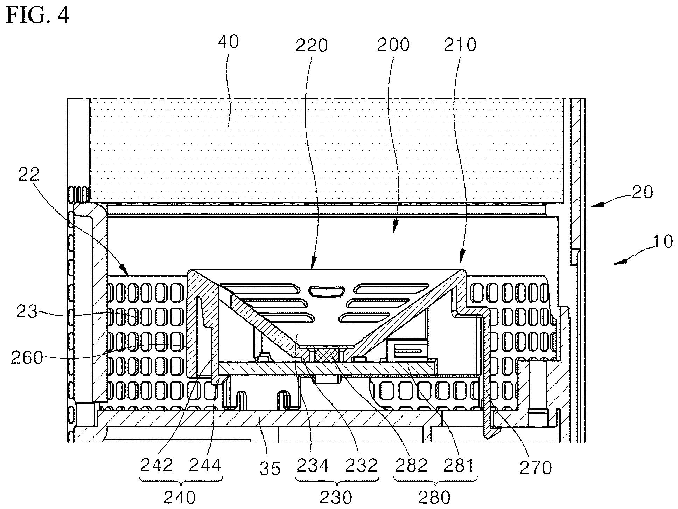

FIG. 4 is a cross-sectional view showing a sterilizer of the first embodiment installed on an inner side of a housing, facing an entrance part;

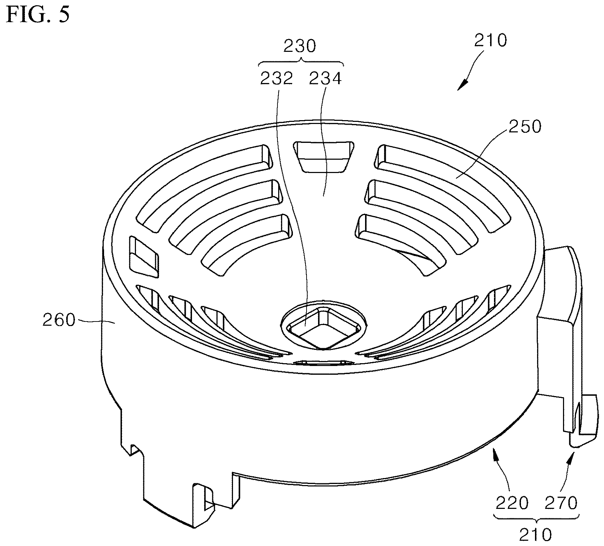

FIG. 5 is a perspective view showing an upper portion of a sterilizing and supporting part of the first embodiment;

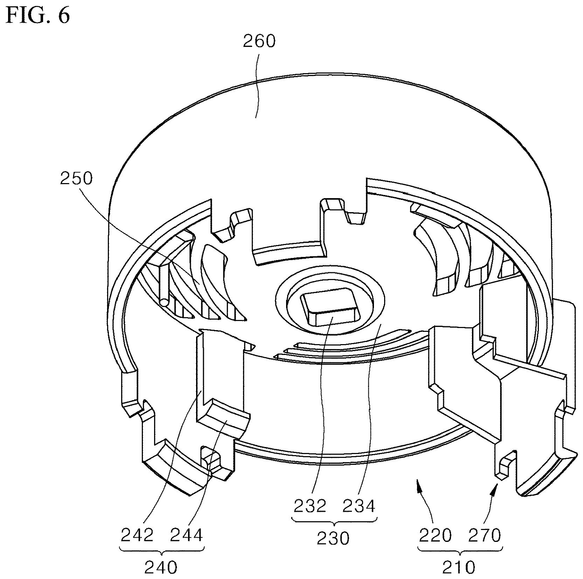

FIG. 6 is a perspective view showing a lower portion of the sterilizing and supporting part of the first embodiment;
FIG. 7 is a cut-away perspective view showing the sterilizing and supporting part of the first embodiment;
FIG. 8 is a view showing the sterilizer of the first embodiment disposed on a lower side of a filter part;
FIG. 9 is a cross-sectional view showing a sterilizer of a second embodiment;
FIG. 10 is a perspective view showing the sterilizer of the second embodiment;
FIG. 11 is a plan view showing the sterilizer of the second embodiment; and
FIG. 12 is a plan view showing a sterilizer of a third embodiment.

### DETAILED DESCRIPTION

The above-described aspects, features and advantages are specifically described hereunder with reference to the accompanying drawings such that one having ordinary skill in the art to which the present disclosure pertains can easily implement the technical concepts of the disclosure. In the disclosure, detailed description of known technologies in relation to the subject matter of the disclosure is omitted if it is deemed to make the gist of the disclosure unnecessarily vague. Below, preferred embodiments according to the disclosure are specifically described with reference to the accompanying drawings. In the drawings, identical reference numerals can denote identical or similar components.

The terms "first", "second" and the like are used herein only to distinguish one component from another component. Thus, the components should not be limited by the terms. Certainly, a first component can be a second component unless stated to the contrary.

When one component is described as being "in an upper portion (or a lower portion)" of another component, or "on (or under)" another component, one component can be disposed on the upper surface (or under the lower surface) of another component, and an additional component can be interposed between another component and one component on (or under) another component.

When one component is described as being "connected", "coupled", or "connected" to another component, one component can be directly connected, coupled or connected to another component. However, it is also to be understood that an additional component can be "interposed" between the two components, or the two components can be "connected", "coupled", or "connected" through an additional component.

Throughout the disclosure, each component can be provided as a single one or a plurality of ones, unless explicitly stated to the contrary.

The singular forms "a", "an" and "the" are intended to include the plural forms as well, unless explicitly indicated otherwise. It should be further understood that the terms "comprise" or "include" and the like, set forth herein, are not interpreted as necessarily including all the stated components or steps but can be interpreted as excluding some of the stated components or steps or can be interpreted as including additional components or steps.

Throughout the disclosure, the terms "A and/or B" as used herein can denote A, B or A and B, and the terms "C to D" can denote C or greater and D or less, unless stated to the contrary.

### [Exterior of portable air purifier]

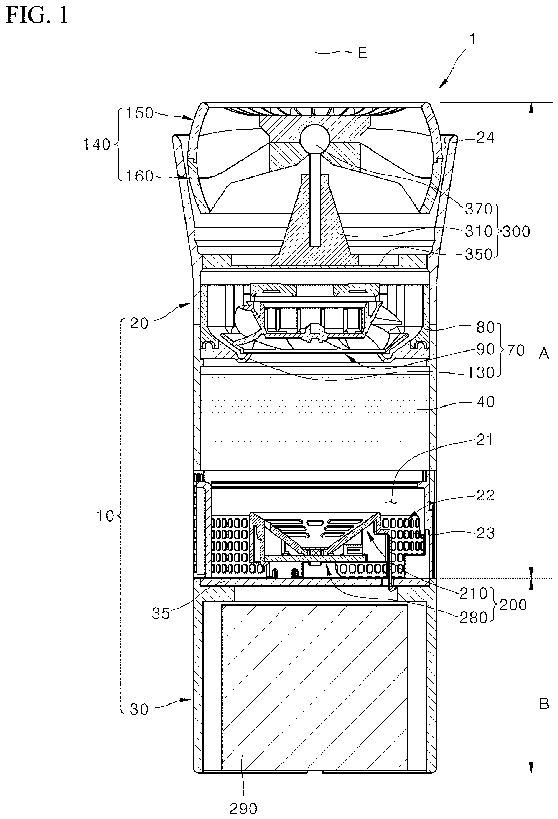

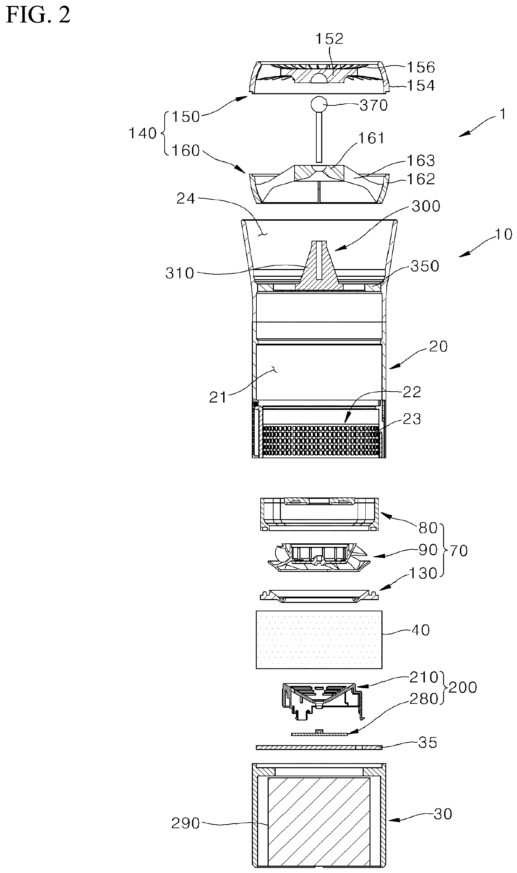

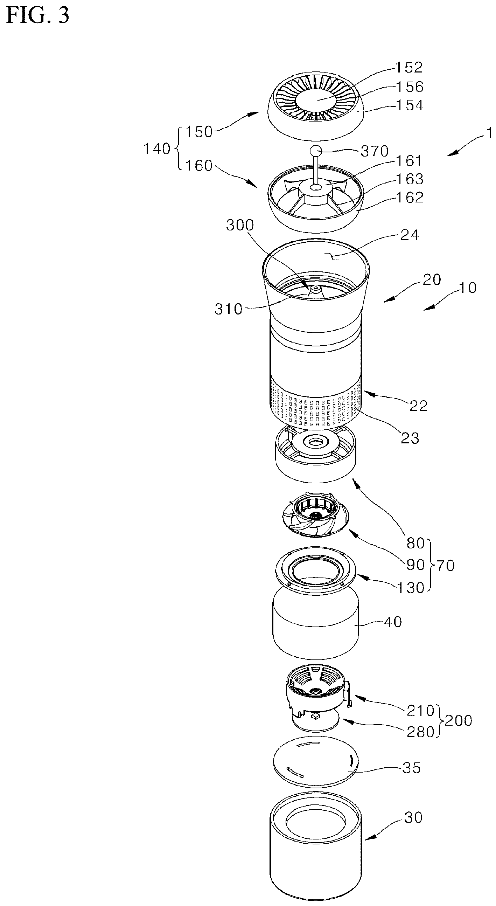

FIG. 1 is a cross-sectional view showing a portable air purifier 1 of a first embodiment, FIG. 2 is a cross-sectional view separately showing the portable air purifier 1 of the first embodiment, and FIG. 3 is a perspective view separately showing the portable air purifier 1 of the first embodiment.

As illustrated in FIG. 1 to 3, the portable air purifier 1 of the first embodiment may have an approximately cylindrical shape. The portable air purifier 1 may include at least one of a housing 10, a filter part 40, a fan module 70, a discharge part 140, a sterilizer 200 and a rotation supporter 300.

The housing 10 may be provided with an entrance part 22, and provided therein with the filter part 40, the sterilizer 200 and the fan module 70. The housing 10 may form an air flow path in an up-down direction. Since the air flow path having a cylindrical shape is formed inside the housing 10, frictional resistance of air moving in the up-down direction may decrease.

Additionally, centers of the entrance part 22, the filter part 40, the sterilizer 200, the fan module 70, the rotation supporter 300 and an outlet 24 may be aligned in the up-down direction along a rotation axis extending line E that passes through the center of the housing 10 in the up-down direction. Accordingly, air moving along the housing 10 from a lower side to an upper side may flow linearly in a perpendicular direction, resulting in a decrease in the length of the flow path of the air, and flow resistance of the air may decrease, thereby improving air purification efficiency.

The rotation axis extending line E may be the same as a rotation center of a fan member included in the fan module 70, and may be aligned with a perpendicular reference line that passes through the center of the hosing 10 and extends in the up-down direction.

When the portable air purifier 1 is placed on a horizontal surface, the perpendicular reference line may be aligned with a perpendicular line or the rotation axis extending line E. The housing 10 may be comprised of a single member, or a plurality of members, for example.

The portable air purifier 1 may be entirely formed into a cylinder that is elongated in the up-down direction and stands. Accordingly, a user may use the portable air purifier 1 in a state where the portable air purifier 1 lies or stands. Further, the portable air purifier 1 may be used in a state where the portable air purifier 1 is installed in a groove part such as a cup holder that is concave downward, in a place such as a rocking vehicle. Thus, the portable air purifier 1 may be reliably disposed in its place.

Directions are defined as follows. Under the assumption that a portion in a direction where the discharge part 140 is disposed with respect to the first case 20 is defined as an upper portion, and a portion in a direction where a second case 30 is disposed with respect to the first case 20 is defined as a lower portion, a "first direction" denotes an up-down direction or an axial direction. The first direction may be referred to as a perpendicular direction. Additionally, a "second direction" denotes a direction perpendicular to the first direction, i.e., a left-right direction, a horizontal direction or a radial direction.

### [Entire structure of portable air purifier]

The portable air purifier 1 of this embodiment may include a housing 10, a filter part 40, a fan module 70 and a sterilizer 200. The portable air purifier 1 may further include a discharge part 140 and a battery 290.

The housing 10 may include a first case 20 and a second case 30. The first case 20 and the second case 30 may form a skeleton of the exterior of the portable air purifier 1. Exteriors of lateral and bottom surfaces of the portable air purifier 1 may be formed by the first case 20 and the second case 30. The first case 20 and the second case 30 may be provided therein with an accommodation space 21. The accommodation space 21 may accommodate the filter part 40, the fan module 70, the sterilizer 200, the rotation supporter 300, electronic components including the battery 290, and the like. The first case 20 and the second case 30 may have enough strength to protect the accommodated components from an external impact, for example.

The filter part 40 may be installed in the accommodation space 21 of the first case 20 and disposed between the fan module 70 and the entrance part 22. That is, the filter part 40 may be disposed under/below the fan module 70 and may purify air suctioned through the entrance part 22 of the portable air purifier 1. The air, which is purified while passing through the filter part 40, may pass through the fan module 70 and the discharge part 140 and may be discharged from an upper portion of the portable air purifier 1.

The filter part 40, disposed on an upper side of the entrance part 22 and configured to purify air suctioned into the entrance part 22 and moving upward, may be installed inside the first case 20 to face the entrance part 22. The filter part 40 may be formed into a cylinder that is elongated in the up-down direction.

Since the filter part 40 is disposed on the upper side of the entrance part 22, the filter part 40 may not be seen from the entrance part 40. Additionally, a predetermined distance may be set between an upper end of the entrance part 22 and a lower end of the filter part 40. The distance may be determined considering an incident angle and an irradiation distance of a light ray for sterilization irradiated from an irradiator 280. Additionally, the distance may also be determined considering reflectivity and performance of a reflector 230.

The filter part 40 may be comprised of a single filter, or when necessary, may be comprised of a plurality of filters in a state of being stacked. The filter part 40 may be further provided with a filter case (not illustrated) for fixing a filter.

The filter case may be fixed to an inside of the first case 20 and may be provided therein with an insertion space for accommodating a filter.

The fan module 70 may be accommodated in the accommodation space 21 of the first case 20 and disposed between the discharge part 140 and the filter part 40. Specifically, the fan module 70 may be disposed between the outlet 24 and the filter part 40. That is, the fan module 70 may be disposed on/over the filter part 40, and the outlet 24, the rotation supporter 300, the discharge part 140 and a rotation guide 400 may be disposed on/over the fan module 70. The fan module 70 may suction air introduced into a lower portion of the filter part 40 through the entrance part 22 and discharge the air from the upper portion of the first case 20.

A rotation center of the discharge part 140 may be aligned with the center of the fan module 70 in the up-down direction. The air suctioned through the entrance part 22 may pass through the filter part 40, the fan module 70, a guide vane 356 and the discharge part 140 consecutively while moving upward, and may be discharged from an upper side of the portable air purifier 1.

In this embodiment, the fan module 70 includes a mixed flow fan, for example. The fan module 70 may suction the air having passed through the filter part 40 in the axial direction and discharge the air in a direction between the axial direction and the radial direction.

The discharge part 140 may be rotatably disposed on an upper side of the first case 20 and may guide a discharge direction of air moved upward through the outlet 24. The rotation supporter 300 may be disposed on/over the first case 20, and the discharge part 140 may be rotatably disposed at/mounted onto the rotation supporter 300. The discharge part 140 may have upper and lower sides that are both open, and air moved to a lower portion of the discharge part 140 through the rotation supporter may be discharge out of the portable air purifier 1 through an upper portion of the discharge part 140.

The sterilizer 200 may be disposed under/below the filter part 40 and fixed to at least one of the first case 20 and the second case 30. The sterilizer 200 may be spaced a predetermined distance apart from the filter part 40, and irradiate a light ray for sterilization toward the filter part 40. The light ray for sterilization irradiated by the sterilizer 200 is harmful to the human body. Accordingly, a position of the sterilizer 200 may be determined such that the light ray for sterilization does not leak out of the portable air purifier 1 through the entrance part 22.

Since the sterilizer 200 may be disposed on an inner side of the housing 10, facing the entrance part 22, an exterior of the housing 10 may not be scaled up despite installation of the sterilizer 200, thereby making it possible to provide a compact-sized portable air purifier 1 and sterilize the filter part 40.

The battery 290 may be installed in the accommodation space 21 provided inside the second case 30 and disposed under/below the sterilizer 200. The battery 290 may supply power for driving the portable air purifier 1.

### [Disposition structure of components of portable air purifier]

The accommodation space 21 provided in the portable air purifier 1 may be divided into a first area A and a second area B. When the accommodation space 21 is divided in the up-down direction, an upper area may be the first area A, and a lower area may be the second area B. The first area A and the second area B may be conceptually divided areas rather than physically divided areas.

In one embodiment, the accommodation space 21 in the first case 20 and the accommodation space 21 in the second case 30, which form the skeleton of the portable air purifier 1, may be the first area A and the second area B respectively.

Components in relation to the suction, purification, discharge and sterilization of air may be disposed in the first area A. That is, the entrance part 22, the filter part 40, the fan module 70, the rotation supporter 300, the discharge part 140 and the sterilizer 200 for sterilizing the filter part 40 may be disposed in the first area A. Accordingly, in the first area A, air may flow from the lower side to the upper side, and a discharge direction of air may be adjusted through the discharge part 140 that is rotatably installed.

The entrance part 22 may be installed in the first case 20, and the entrance part 22 may be provided with a plurality of entrance holes 23 as a passage for suctioning air. The outlet 24 as a passage for discharging air purified in the first area A, and the discharge part 140 rotatably disposed at/mounted onto the rotation supporter 300 may be disposed on/over the first case 20. Accordingly, the first case 20 may be provided therein with an air flow path connecting the filter part 40, the fan module 70 and the discharge part 140.

Additionally, the sterilizer 200 may be disposed in an inner space of the housing 10, facing the entrance part 22. Accordingly, the housing 10 may not be scaled up to install the sterilizer 200, and space availability may improve.

That is, the entrance part 22, the filter part 40, the fan module 70, the discharge part 140, the sterilizer 200, and the outlet 24 may be installed in the first area A. A passage for allowing air, suctioned into the portable air purifier 1, to pass through the air purifier may be formed in the first area A.

Components that do not directly relate to a flow of air for air purification may be disposed in the second area B. That is, a controller including a PCB, the battery 290 and the like may be installed in the second area B.

In this embodiment, the housing 10 may be formed into a cylinder that has a vertical length greater than a lateral length. The first area A in the upper portion may have a vertical length greater than a vertical length of the second area B in the lower portion. That is, in the portable air purifier 1 that stands vertically, the first area A in the upper portion may be greater than the second area B in the lower portion.

The discharge part 140 may be rotatably disposed at/mounted onto the rotation supporter 300. Accordingly, a discharge direction of air purified in the upper portion of the portable air purifier 1 may be readily adjusted, and air purified in the portable air purifier 1 may reach the face of the user more readily.

When the portable air purifier 1 is placed and used on a bottom surface placed further downward than the face of the user, the portable air purifier 1 may stand vertically rather than lie transversely to allow more air, purified in the portable air purifier 1, to reach the face of the user.

To this end, when air is discharge from the upper portion of the portable air purifier 1 through the discharge part 140 rotated in a predetermined direction in a state where the portable air purifier 1 stands vertically, more air purified in the portable air purifier 1 may reach the face of the user.

Since the sterilizing and supporting part 210 is arranged along a perimeter of the irradiator 280, a light ray for sterilization aiming at the filter part 40 may be blocked from being irradiated out of the housing 10 through the entrance part 22. The irradiator 280 may be provided with a reflector 230 around an outer perimeter thereof, and a light ray for sterilization irradiated from the irradiator 280 to the filter part 40 may be prevented from being irradiated out of the entrance part 22 and may be guided and irradiated to a lower side of the filter part 40 by the reflector 230.

### [Specific configuration of component of portable air purifier]

A portable air purifier 1 of the first embodiment may include at least one of a housing 10, a filter part 40, a fan module 70, a discharge part 140, and a sterilizer 200. The portable air purifier 1 in the first embodiment may further include a rotation supporter 300 and a battery 290.

### [First case]

The housing 10 may include a first case 20 and a second case 30. The first case 20 may be provided with an accommodation space 21 therein and an entrance part 22 for suctioning air on a lateral surface of a lower portion thereof. The first case 20 may be formed into a cylinder and have upper and lower sides that are open. The first case 20 may be comprised of a single component, or when necessary, may be comprised of a plurality of members. A first case 20 in the first embodiment is comprised of a plurality of members, and each member may be fitted-coupled, coupled using an adhesive or welded and may be coupled in various ways such as a connection using a fastening member 195 including a bolt and the like.

Air may be suctioned through the lateral surface of the lower portion of the first case 20 and discharged through an upper side of the first case 20. To this end, the entrance part 22 provided with entrance holes 23 may be disposed along a perimeter of a lower portion of the first case 20. The first case 20 may be provided with an outlet 24 for discharging air, on the upper side thereof.

In a state in which the entrance part 22 for suctioning air is disposed around an outer perimeter of the first case 20, a filter part 40 may be disposed on an upper side in a way that the filter part 40 is spaced from the entrance part 22. Accordingly, air may move uniformly across an entire surface of the filter part 40.

The entrance part 22 may be provided with a plurality of entrance holes 23. The entrance hole 23 may be disposed at a slant in an oblique line shape, and when necessary, formed into an inequality sign. The entrance hole 23 may be modified in various ways. For example, to increase a flow rate of air flowing into the filter part 40, the entrance hole 23 may be additionally formed on a lateral surface of the housing 10, on which the filter part 40 is disposed.

The housing 10 may be modified in various ways. For example, the hosing 10 may be comprised of three or more members.

### [Second case]

The second case 30 may connect to the lower portion of the first case 20, and may be modified in various ways within the technical scope in which the second case is provided therein with a space in which electronic components including a battery 290 is installed.

At least one of the first case 20 and the second case 30 may be formed into a cylindrical case. The first case 20 and the second case 30 may both be formed into a cylinder or the second case 30 may only be formed into a cylinder. When necessary, the first case 20 may only be formed into a cylinder.

In the case of a second case 30 having a cylindrical shape and extending in the up-down direction, the user may readily hold an outer perimeter of the second case 30 in the hand, and the second case 30 may be easily held in a vehicle's cup holder provided with a groove part ordinarily having a circular cross section.

In the case of a first case 20 having a cylindrical shape, friction, caused as a result of contact between air, which moves upward while passing through the first case 20, and an inside of the first case 20 having a curved shape, may be reduced, thereby enabling the air to flow more smoothly.

Additionally, an air flow path may be formed inside the first case 20, and no air flow path may be formed inside the second case 30. Accordingly, air may be smoothly suctioned and discharged through the first case 20 even when the second case 30 is held in the cup holder or the user's hand, thereby ensuring improvement in usability and convenience.

### [Inner housing]

The inner housing 35 for supporting a sterilizer 200 may be disposed on the second case 30. The inner housing 35 may be formed into a plate and may support a lower portion of the sterilizer 200. An inner housing 35 in the first embodiment may be formed into a circular plate, and may shield an upper side of the second case 30.

The inner housing 35 may be fixed to at least one of the first case 20 and the second case 30 and provided with a hole for fixing a support bracket 270 of the sterilizer 200.

The inner housing 35 may be disposed between the first case 20 and the second case 30 and may shield the lower portion of the first case 20. Additionally, the inner housing 35 may be disposed on a lower side of the sterilizer 200, and may be modified in various ways within the technical scope in which the inner housing connects to the housing 10 and movement of the inner housing is restricted.

Air suctioned into the first case 20 through the entrance part 22 may be blocked from moving to the second case 30 by the inner housing 35 such that a flow rate of air moving to the fan module 70 increases, thereby ensuring improvement in air purification performance of the portable air purifier 1.

### [Filter part]

The filter part 40 may be disposed inside the first case 20 and may be modified in various ways within the technical scope in which the filter part purifies air suctioned into the entrance part 22. A filter part 40 in the first embodiment maybe formed into a cylinder.

The first case 20 may be formed into a circular pipe, and the filter part 40 installed in the first case 20 may be formed into a cylinder in contact with the inside of the first case 20. Accordingly, impurities in air passing through the first case 20 may be removed effectively.

A transverse cross section of the filter part 40 may have a circular shape, the filter part 40 may have the largest area in the first case 20. Additionally, the filter part 40 may be formed into a cylinder, and when upper and lower ends of cloth of the filter part 40 are cut, pressure loss may be minimized, and performance of the filter part 40 may be maximized.

An outer diameter of the filter part 40 may be greater than a diameter of an entrance through which air is suctioned into the fan module 70. Accordingly, volume of the filter part 40 may be maximized.

In this embodiment, the filter part 40, the fan module 70, the sterilizer 200, the discharge part 140 may be disposed along the housing 10 in the up-down direction, and air may also flow in the up-down direction. That is, air may flow linearly in a direction the same as the direction in which the filter part 40, the fan module 70 and the discharge part 140 are disposed, as a result of operation of the fan module 70.

When air flows linearly, resistance against an air flow may decrease, and air may flow more smoothly. Thus, a sufficient amount of air may be suctioned and discharged by the fan module 70, and air purification performance of the portable air purifier 1 may improve.

An antimicrobial HEPA filter may be used as a filter part 40 in the first embodiment. When power is not properly supplied to the sterilizer 200, an irradiator 280 may not operate, and the filter part 40 may not be sterilized correctly. To solve the problem, a HEPA filter to which an antimicrobial agent is applied may be used as the filter part 40.

### [Fan module]

The fan module 70 may be disposed between the filter part 40 and the outlet 24, and may be modified in various ways within the technical scope in which the fan module rotates a fan to blow air toward the outlet 24.

When a circular mixed flow fan module is applied to the fan module 70, the first case 20 may not be scaled-up despite fixation or a coupling of the fan module 70 since the shape of the fan module 70 matches or corresponds to the cylindrical shape of the inside of the first case 20, thereby making a product smaller. Additionally, when the portable air purifier 1 according to the present disclosure is applied to a vehicle, the portable air purifier 1 may be small enough to fit into a cup holder.

Since a circular mixed flow fan module is applied to the fan module 70, a small-sized upward discharge-type air purifier, which ensures maximized hydrodynamic performance, may be provided. A fan of the fan module 70 may be comprised of a mixed flow fan, and an inner structure of the fan module 70 may change to mount the mixed flow fan.

A fan member 90 according to the disclosure may rotate as a result of operation of a motor. A rotation shaft of the motor configured to rotate the fan member 90 may only connect to the fan member 90. A rotor may be installed in the fan member 90, and a stator may be installed in the fan housing 80 the rotation of which is restricted. Since a magnetic field of the stator changes, the shaft connected to the fan member 90 may rotate along with the rotor such that the rotor and the fan member 90 rotate around the stator. The configuration of the motor for rotating the fan member 90 is well known. Thus, detailed description in relation to this is omitted.

A fan module 70 in the first embodiment may include a fan housing 80, a fan member 90, and a fan base 130.

The fan housing 80 may be fixed to the inside of the first case 20, and may be modified in various ways within the technical scope in which the fan housing is provided therein with a space for rotating the fan member 90.

The fan member 90 may be rotatably installed in the fan housing 80, and may be modified in various ways within the technical scope in which the fan member moves air toward the discharge part 140.

In one embodiment, a mixed flow fan may be used as the fan member 90 but not limited. Another type of fan may also be used as the fan member 90 according to the disclosure.

The fan base 130 may be coupled to a lower side of the fan housing 80, and may be modified in various ways within the technical scope in which the fan base guides air having passed through the filter part 40 into the fan member 90.

The fan base 130 may be disposed between the filter part 40 and the fan member 90. An edge of the fan base 130 may have a shape corresponding to a shape of an edge of the filter part 40. For example, when the filter part 40 has a cylindrical shape and the edge of the filter part 40 has a circular shape, the fan base 130 may be formed into a ring provided with a hollow hole/may be installed in a ring shape provided with a hollow hole.

### [Discharge part]

As illustrated in FIGS. 1 to 3, the discharge part 140 may be rotatably disposed at/mounted onto a rotation supporter 300, and may be modified in various ways within the technical scope in which the discharge part adjusts a discharge direction of air having passed through the fan module 70. The discharge part 140 according to the disclosure may be rotatably disposed at/mounted onto a sphere-shaped ball joint 370 installed in the rotation supporter 300 and may rotate smoothly.

The discharge part 140 may be open in the up-down direction and may rotatably connect to the rotation supporter 300. Accordingly, the discharge part 140 may adjust a discharge direction of air having passed through the fan module 70. A discharge part 140 in the first embodiment may include a first discharge part 150 and a second discharge part 160.

The first discharge part 150 may be disposed on one side of the ball joint 370 (an upper side in FIG. 2), and may be modified in various ways within the technical scope in which the first discharge part is provided with a plurality of vanes 156 configured to guide discharge of air. A first discharge part 150 in the first embodiment may include a first discharge core 152, a first discharge body 154, and a vane 156.

The first discharge core 152 may surround an upper side of the sphere shape of the ball joint 370 and may be disposed on an upper side of a core member 310. Additionally, the first discharge body 154 may surround an outside of the first discharge core 152 in a ring shape, and an outside of the first discharge body 154 may be formed into a curved surface.

Since the first discharge core 152 and the first discharge body 154 may be connected by a plurality of vanes 156, the first discharge core 152, the first discharge body 154 and the vanes 156 may rotate together.

The second discharge part 160 may be disposed on the other side of the ball joint 370 (a lower side in FIG. 2) and connected to the first discharge part 150, and may be modified in various ways within the technical scope in which the second discharge part rotates around the ball joint 370 together with the first discharge part 150. A second discharge part 160 in the first embodiment may include a second discharge core 161, a second discharge body 162, and a discharging and supporting part 163.

The second discharge core 161 may surround a lower side of the sphere shape of the ball joint 370 and may be disposed on a lower side of the first discharge core 152. Additionally, the second discharge body 162 may surround an outside of the second discharge core 161 in a ring shape, and an outside of the second discharge body 162 may be formed into a curved surface.

Since the second discharge core 161 and the second discharge body 162 may be connected by a plurality of discharging and supporting parts 163, the second discharge core 161, the second discharge body 162, and the discharging and supporting parts 163 may rotate together.

### [Rotation supporter]

The rotation supporter 300 may be modified in various ways within the technical scope in which the rotation supporter rotatably supports the discharge part 140 disposed in the outlet 24 of the housing 10. A rotation supporter 300 in the first embodiment may include a core member 310, a core supporter 350 and a ball joint 370.

The core member 310 may be disposed on a lower side of the discharge part 140 and extended toward the discharge part 140. The core member 310 may be disposed on the lower side of the discharge part 140 configured to adjust a discharge direction of air and extended from a center of the outlet 24 toward the discharge part 140.

An outside of the core member 310 may be formed into a curved surface, and a transverse cross section of the core member 310 may gradually become narrow from a lower side of the core member 310, connected to the core supporter 350, toward the ball joint 370. Accordingly, resistance of air moving from the lower side of ... to the upper side thereof may be minimized.

Alternatively, the outside of the core member 310 may be formed into a curved surface, and may be modified in various ways. For example, a transverse cross section of the core member remains constant or changes from the lower side of the core member, connected to the core supporter 350, toward the ball joint 370.

The core member 310 may have a circular cone shape, and the transverse cross section of the core member may gradually decrease further toward the upper side of the core member.

The core supporter 350 may be modified in various ways within the technical scope in which the core supporter supports a lower portion of the core member 310 and is fixed to the housing 10.

A vane disposed at/installed in the core supporter 350 may be modified in various ways. For example, the vane may be extended radially or installed in a spiral shape with respect to the core member 310.

The ball joint 370 may be modified in various ways within the technical scope in which a lower side of the ball joint is coupled to the core member 310 such that movement of the ball joint 370 is restricted, and an upper side of the ball joint is inserted into the discharge part 140 and rotatably supports the discharge part 140. The ball joint 370 may have a sphere-shaped end, may be disposed inside the discharge part 140 and may rotatably support the discharge part 140.

### [Sterilizer]

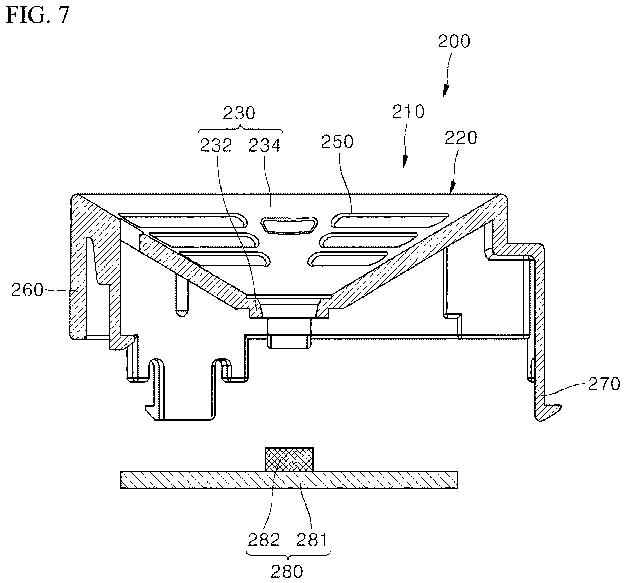

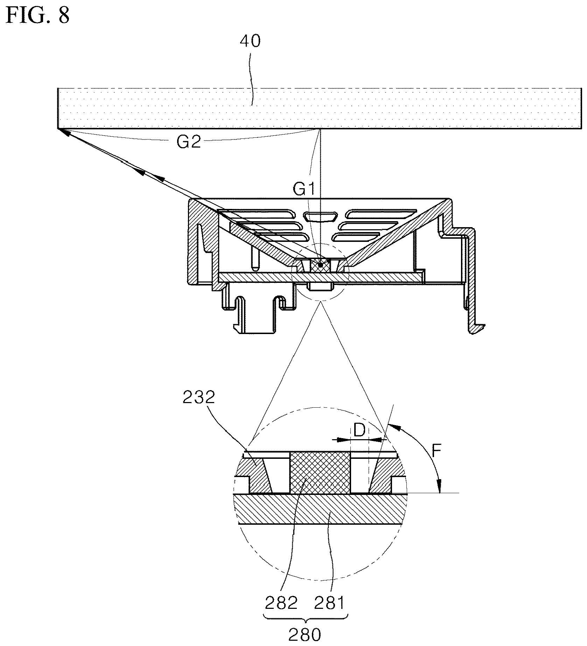

FIG. 7 is a cut-away perspective view partially showing the sterilizing and supporting part 210 in the first embodiment, and FIG. 8 is a view showing the sterilizer 200 in the first embodiment disposed on a lower side of a filter part 40.

As illustrated in FIGS. 1, 7 and 8, the sterilizer 200 may be disposed on a lower side of the filter part 40 and disposed at the same height as that of the entrance part 22. Since the sterilizer 200 and the entrance part 22 are disposed at the same height, the air purifier according to the present disclosure may ensure improvement in space availability unlike an air purifier in which a sterilizer 200 is disposed on an upper side of an entrance part 22, and the housing 10 according to the disclosure may be disposed at a low position unlike a housing 10 of the air purifier in which the sterilizer 200 is disposed on the upper side of the entrance part 22. The sterilizer 200 may be modified in various ways within the technical scope in which the sterilizer irradiates a light ray for sterilization to sterilize the filter part 40. The sterilize 200 in the first embodiment may include a sterilizing and supporting part 210 and an irradiator 280.

The sterilizing and supporting part 210 may be fixed to the housing 10 while supporting the irradiator 280. The sterilizing and supporting part 210 may be modified in various ways within the technical scope in which the sterilizing and supporting part is disposed around an outer perimeter of the irradiator 280. The sterilizing and supporting part 210 in the first embodiment may be fixed to at least one of the first case 20 and the second case 30. The sterilizing and supporting part 210 may include a support body 220 and a support bracket 270.

The support body 220 may be disposed around the outer perimeter of the irradiator 280, and may be modified in various ways within the technical scope in which the support body reflects a light ray for sterilization irradiated from a sterilizing light source 282 to the filter part 40. A support body 220 in the first embodiment may include at least one of a reflector 230, an inner supporter 240, a heat dissipating hole part 250 and a blocking body 260.

The reflector 230 may be concave toward the filter part 40 and provided with the sterilizing light source 282 of the irradiator 280 at a center thereof. The reflector 230 may reflect light irradiated from the sterilizing light source 282 of the irradiator 280 toward the filter part 40.

The reflector 230 may be disposed on an inner side of the housing 10, facing the entrance part 22. Since ultraviolet rays used as the sterilizing light source 282 are harmful to the human body, the support body 220 including the reflector 230 may be installed to prevent a light ray for sterilization from being irradiator out of the housing 10. The reflector 230 may be installed to block a light ray for sterilization from being irradiated to the housing 10, which is an injection mold, except for the filter part 40, or from leaking out of the housing 10 through the entrance part 22. The reflector 230 may be designed considering an incident angle of a light ray for sterilization and a surface area of the filter part 40.

The reflector 230 may be formed into a funnel, an upper side of which is open, and an upper end of the reflector 230 may be disposed on the same line as an upper end of the entrance part 22 or disposed higher than the upper end of the entrance part 22. Accordingly, a light ray for sterilization irradiated from the sterilizing light source 282 may be prevented from being irradiated out of the entrance part 22.

Additionally, an angle of the reflector 230 may be designed not to limit a light ray for sterilization toward an edge of the filter part 40. A reflector 230 in the first embodiment may include a first reflecting body 232 and a second reflecting body 234.

The first reflecting body 232 may be disposed along a perimeter of the sterilizing light source 282 and spaced a predetermined distance D apart from the sterilizing light source 282.

The second reflecting body 234 may extend upward from the first reflecting body 232 at a slant. A second reflecting body 234 in the first embodiment may be formed into a funnel an upper side of which is open, and an inner diameter of the second reflecting body 234 may gradually increase from the first reflecting body 232 toward the filter part 40.

A reflecting and coating layer may be formed on a surface of the reflector 230. The surface of the reflector 230 which is an injection mold may be coated with a material such as aluminum, chrome and the like having high reflectivity such that the reflector 230 serves as a reflecting plate.

The first reflecting body 232 facing the sterilizing light source 282 may only be provided with a coating layer. When necessary, the second reflecting body 234 may also be provided with a coating layer. When the reflector 230 is coated with aluminum, reflectivity may be 90 %.

The reflecting surface of the first reflecting body 232, facing the sterilizing light source 282, may be installed while forming a predetermined angle F with a horizontal line. A predetermined angle F of a reflecting surface in the first embodiment may be a slant angle of 805 degrees. Accordingly, a light ray for sterilization irradiated from the sterilizing light source 282 may be directly irradiated to the edge of the filter part 40, or may reflect off the first reflecting body 232 and then be irradiated to the edge of the filter part 40, thereby minimizing loss of the light ray for sterilization. Since the reflector 230 is installed as describe above, an average illumination may improve to 6%, and a minimum illumination may improve to 26% with respect to UVC-LED 10mW.

A predetermined distance D between a sterilizing light source 282 and a first reflecting body 232 in the first embodiment may be 1.4 mm to 5 mm. Suppose that a distance between the sterilizing light source 282 and the filter part 40 is referred to as a first length G1 and that a radius of a lower surface of the filter part 40 is referred to as a second length G2. In this case, a second length G2 in the first embodiment may be 1.8 to 2.2 times greater than a first length G1.

The inner supporter 240 may be modified in various ways within the technical scope in which the inner supporter extends to a lower side of the reflector 230 and supports the printed circuit board 281 of the irradiator 280. An inner supporter 240 in the first embodiment may include an inner bracket 242 extended to the lower side of the reflector 230, and a held hook 244 bent inward from a lower side of the inner bracket 242 to the irradiator 282 and held at an edge of the irradiator 280.

A plurality of inner supporters 240 may be provided and extended to the lower side of the reflector 230. The inner supporter 240 may operate like a hook, and may be held at an outer edge of the printed circuit board 281. Accordingly, the printed circuit board 281 may be fixed to an inside of the support body 220.

The heat dissipating hole part 250 may form a plurality of holes at the reflector 230. Since air moves through the heat dissipating hole part 250, the heat dissipating hole part 250 may air-cool the irradiator 280. The heat dissipating hole part 250 may be provided with a plurality of long holes having a circular arc shape around the sterilizing light source 282. In the heat dissipating hole part 250, a slit hole extended in a circular arc form may be disposed around the sterilizing light source 282. As a result of the installation of the heat dissipating hole part 250, the printed circuit board 281 of the irradiator 280 disposed on a lower side of the irradiator 280 may be air-cooled, thereby improving heat dissipation performance of the sterilizing light source 282 using a UVC LED.

The blocking body 260 may extend from an outer edge of the reflector 230 to the lower side thereof. The blocking body 260 may be formed into a cylinder a lower side of which is open, and centers of the blocking body 260 and the entrance part 22 may be aligned with each other. Since the blocking body 260 is provided, the blocking body 260 may block movement of the sterilizing light source 282 although the sterilizing light source 282 is irradiated from the irradiator 280 to the lower side of the reflector 230. Accordingly, when a light ray for sterilization is about to be irradiated out of the housing 10 through the entrance part 22, the reflector 230 may block the light ray for sterilization primarily, and the blocking body 260 may block the light ray for sterilization secondarily.

The support bracket 270 may be modified in various ways within the technical scope in which the support bracket extends to a lower side of the support body 220 and is fixed to the housing 10. The support bracket 270 may extend to the lower side of the support body 220 and may be fixed to at least one of the first case 20 and the second case 30 or may be fixed to the inner housing 35 shielding the upper side of the second case 30.

A support bracket 270 in the first embodiment may be coupled to the inner housing 35 having a plate shape in a hook form. Accordingly, the sterilizer 200 may be installed and disassembled easily, and time and costs spent for maintenance and repairs of the sterilizer 200 may decrease.

The irradiator 280 may be disposed on the lower side of the filter part 40 and disposed at a height where the irradiator 280 overlaps the entrance part 22, and may irradiate a light ray for sterilization to the filter part 40. Additionally, movement of the irradiator 280 may be restricted by the sterilizing and supporting part 210 and may be on a perpendicular reference line that passes through a radius-wise center of the entrance part 22 in the up-down direction. The perpendicular reference line and the rotation axis extending line may form the same straight line. Further, the centers of the first case 20, the filter part 40, the fan module 70 and the irradiator 280 may be aligned in the up-down direction.

When the filter part 40 is disposed on an upper side of the irradiator 280, a sterilizing light source 282 supplying a relatively small amount of light may sterilize an entire surface area of the lower end of the filter part 40, thereby reducing costs incurred for manufacturing, maintenance and repairs.

The irradiator 280 may be modified in various ways within the technical scope in which the irradiator is disposed at a position higher than or the same as that of the upper end of the entrance part 22. An irradiator 280 in the first embodiment may include a printed circuit board 281 and a sterilizing light source 282. The printed circuit board 281 may be fixed to an inside of the sterilizing and supporting part 210, and the sterilizing light source 282 configured to irradiate a light ray for sterilization may be disposed on an upper side of the printed circuit board 281 or inside the printed circuit board 281.

The printed circuit board 281 may be held by the inner supporter 240 included in the support body 220 such that movement of the printed circuit board 281 is restricted. The sterilizing light source 282 disposed on the printed circuit board 281 may be disposed on the lower side of the filter part 40 and may irradiate a light ray for sterilization at an incident angle set to face an upper side on which the filter part 40 is disposed. Since radius-wise centers of the sterilizing light source 282 and the filter part 40 are aligned in the up-down direction, a light ray for sterilization may be uniformly irradiated to a lower portion of the filter part 40.

The sterilizing light source 282 may be a UVC LED, and a variety of sterilizing devices may be used within the technical scope in which the sterilizing light source sterilizes germs in the filter part 40.

Since the reflector 230 is disposed along the perimeter of the sterilizing light source 282 although the sterilizing light source 282 of the sterilizer 200 and the entrance part 22 are disposed at the same height, a light ray for sterilization may be prevented from being irradiated out of the first case 20 through the entrance part 22.

In the related art, an incident angle of the UVC LED configured to irradiate a light ray for sterilization is limited. Accordingly, when a proper distance between the filter part 40 and the UVC LED is not ensured, the edge of the filter part 40 may not be sterilized by the UVC LED.

According to the present disclosure, thanks to the reflector 230 and the reflective coating of the reflector 230, a light ray for sterilization may be uniformly irradiated to the lower portion of the filter part 40, thereby ensuring improvement in sterilization performance. Since the reflector 230 may be disposed around the outer perimeter of the irradiator 280 and formed into a funnel, the reflector 230 may reflect a light ray for sterilization to the filter part 40. Additionally, since reflectivity increases thanks to the reflective coating of the reflector 230, an incident angle and an irradiation distance of a light ray for sterilization may be ensured even in a limited space.

### [Air flow and sterilization of portable air purifier]

The entrance part 22 for suctioning external air may be disposed around the outer perimeter of the first case 20. Accordingly, air outside the first case 20 may move into the first case 20 through the entrance part 22 such that a flow rate of suctioned air increases.

As a result of operation of the fan module 70, air outside the portable air purifier 1 may flow into the portable air purifier 1. In this case, the air outside the portable air purifier 1 may form a spiral-shaped air flow rotating around an outer perimeter of the inner supporter 240 while passing through the entrance holes 23.

The air, which flows into the first case 20 and moves upward while rotating in a spiral shape, may move up toward the filter part 40, rotating a perimeter of the sterilizer 200. Additionally, the air having passed through the filter part 40 may move to an upper side of the housing 10 through the fan module 70.

While the air passes through the filter part 40, physical particles such as dust, fine dust, ultra fine dust and the like, chemical substances such as odorant particles, harmful gases and the like, microorganisms such as germs, viruses and the like in the air may be filtered.

Since the filter part 40, the fan module 70 and the rotation supporter 300 are disposed on one straight line in the up-down direction, flow loss may be minimized and air may be effectively suctioned and filtered.

Air suctioned into the fan module 70 may be discharged from an upper side of the fan module 70 and may move into the discharge part 140. Since the discharge part 140 rotates within predetermined angles, a direction of discharged air may be adjusted depending on an angle at which the discharge part 140 is installed.

Additionally, an inside of the discharge part 140 may form a concave groove part. Accordingly, discharge resistance of air, the direction of which is changed through the discharge part 140, may decrease. Further, since the filter part 40, the fan module 70 and the discharge part 140 are disposed on one straight line in the up-down direction, flow loss of air may be minimized, air may be effectively suctioned and filtered, and purified air may be effectively discharged.

Germs in the air may be collected in the filter part 40, and a light ray for sterilization irradiated from the sterilizing light source 282 may be irradiated to the lower portion of the filter part 40, A light ray for sterilization may be directly irradiated to the filter part 40 or may reflect off the reflector 230 and then be irradiated to the lower portion of the filter part 40 to sterilize the filter part 40.

The sterilizing light source 282 may use a light ray having a wavelength of 275 nm that shows high sterilization efficiency as a light ray for sterilization, and considering a UV radiation range on the surface of the filter part 40 using a HEPA filter and reliability of a filter, the first length G1 as an irradiation distance may be set to 17.7 mm or so.

As the filter part 40 and the sterilizing light source 282 become closer, sterilization performance may improve, but after the filter part 40 is used for a long time, degradation of the filter part 40 may cause damage to an exterior of the filter part 40 or particle removal performance of the filter part 40 may deteriorate. Additionally, when an optimal distance between the filter part 40 and the sterilizing light source 282 is not maintained, an amount of light reaching the edge of the filter part 40 may decrease, and sterilization performance may deteriorate. With the sterilizer 200 according to the present disclosure, surface illumination may be ensured even for the edge of the filter part 40, and reliability of the filter part 40 may be ensured.

When a portable air purifier 1 provided with the sterilizer 200 is installed in a vehicle, germs and viruses in the vehicle may be attached to the filter part 40 and then a light ray for sterilization may be irradiated to eliminate the germs attached to the filter part 40.

Bacteria and viruses in polluted air suctioned into an inlet of a vehicle may be collected using an electrostatic force of the filter part 40 using a HEPA filter. Then a light ray for sterilization may be irradiated to eliminate the bacteria and viruses collected in the filter part 40.

### [Second embodiment]

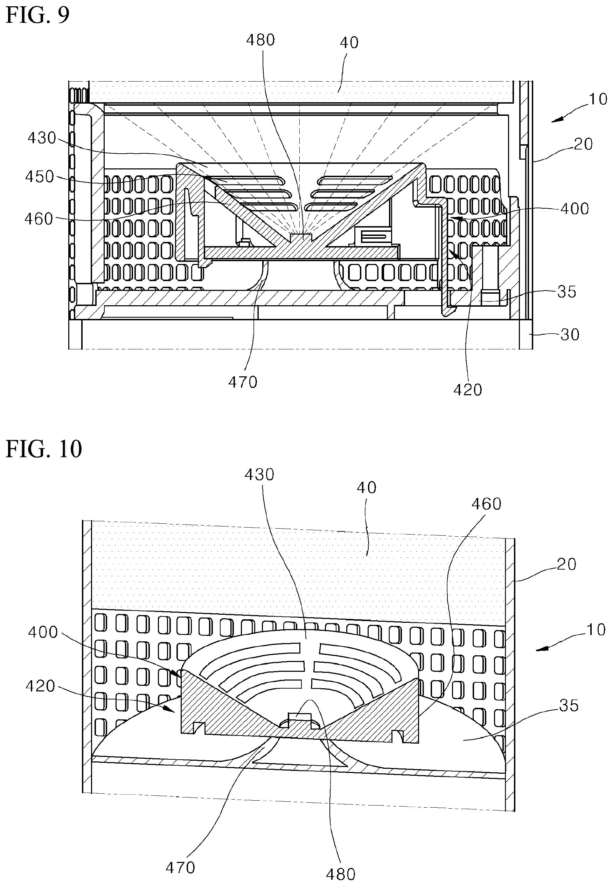

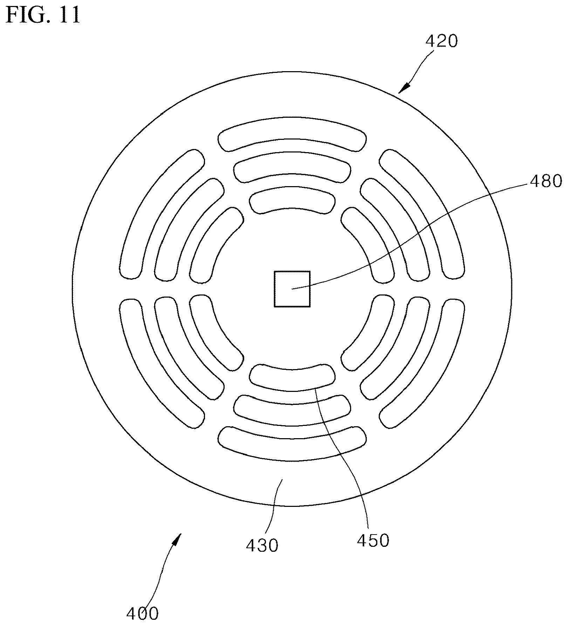

FIG. 9 is a cross-sectional view showing a sterilizer 400 of a second embodiment, FIG. 10 is a perspective view showing the sterilizer 400 of the second embodiment, and FIG. 11 is a plan view showing the sterilizer 400 of the second embodiment.

As illustrated in FIGS. 9 to 11, the sterilizer 400 of the second embodiment may include a sterilizing and supporting part 410 and an irradiator 480.

The sterilizing and supporting part 410 may support a lower portion of the irradiator 480, may be disposed on an inner side of a housing 10, facing an entrance part 22, and may include a support body 420 and a support part 470.

A support body 420 in the second embodiment may include a reflector 230, a heat dissipating hole part 450, and a blocking body 460. The reflector 430 may be formed into a funnel an upper side of which is open, and may be provided with the radiator 480 configured to irradiate a light ray for sterilization at a concave center thereof.

Since a plurality of heat dissipating hole parts 450, forming a slit hole circumferentially, is disposed at the reflector 430, the heat dissipating hole parts 450 may air-cool the irradiator 480 fixed to the support body 420. The reflector 430 may be provided with the blocking body 460 having a pipe shape around an outer perimeter thereof.

The support body 420 may be supported by the support part 470. The support part 470 may be modified in various ways within the technical scope in which the supporting part protrudes upward from a center of an inner housing 35 and supports a lower portion of the support body 420. The support part 470 may be disposed at a center of the entrance part 22 in a radial direction thereof, and a transverse cross section of the support part 470 may have a circular shape to reduce friction between the support part 470 and air.

The support part 470 may be formed into a pillar that protrudes upward from the center of the inner housing 35. The support part 470 may be formed into a cylinder or a circular cone. A support part 470 in the second embodiment may have a transverse cross section that gradually becomes narrow from a lower side to an upper side, and since the support part 470 is disposed at a center of a first case 20, where the entrance part 22 is formed, friction between the support part 470 and air may be minimized.

The transverse cross section of the support part 470 may have a circular shape, and air suctioned through the entrance part 22 may move to an upper side on which a filter part 40 is disposed while rotating an outside of the support part 470. That is, the sterilizer 400 may be disposed in a central portion of the first case 20, and air suctioned through the entrance part 22 may move up while rotating around an outer perimeter of the sterilizer 400. Accordingly, flow resistance of the sterilizer 400 may decrease.

A radius-wise center of the support part 470, a rotation center of a fan member 90 and a radius-wise center of the filter part 40 may be on one straight line in the perpendicular direction. In this case, resistance against a flow of air moving from the lower side to the upper side may decrease, and air may flow more smoothly. Thus, the portable air purifier 1 may ensure improvement in air purification performance.

### [Third embodiment]

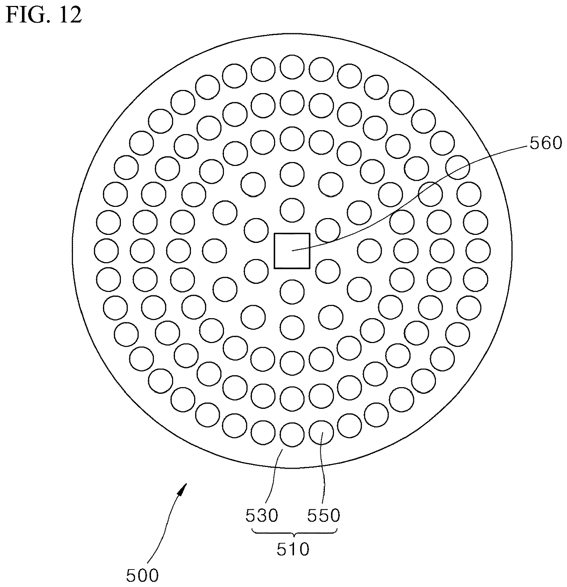

FIG. 12 is a plan view showing a sterilizer 500 of a third embodiment. As illustrated in FIG. 12, the sterilizer 500 of the third embodiment may include a sterilizing and supporting part 510 and an irradiator 560. The sterilizing and supporting part 510 may include a reflector 530 and a heat dissipating hole part 550, and the irradiator 560 configured to irradiate a light ray for sterilization may be disposed at a concave center of the reflector 530.

The reflector 530 may be provided with a plurality of heat dissipating hole parts 550 forming a circular hole. Accordingly, the heat dissipating hole parts 550 may air-cool the irradiator 560 fixed to the sterilizing and supporting part 510. The heat dissipating hole part 550 may be formed into various shapes including a triangle, a polygon and the like, and may be modified in various ways within the technical scope in which the heat dissipating hole part air-cools the irradiator 560.

The embodiments are described above with reference to a number of illustrative embodiments thereof. However, the present disclosure is not intended to limit the embodiments and drawings set forth herein, and numerous other modifications and embodiments can be drawn by one skilled in the art without departing from the technical concept of the disclosure. Further, the effects and predictable effects based on the configurations in the disclosure are to be included within the range of the disclosure though not explicitly described in the description of the embodiments.

**[Description of Reference Numeral]**

| | | | |
|---|---|---|---|
| 1: | Portable air purifier | 240: | Inner supporter |
| 10: | Housing | 242: | Inner bracket |
| 20: | First case | 244: | Held hook |
| 21: | Accommodation space | 250: | Heat dissipating hole part |
| 22: | Entrance part | 260: | Blocking body |
| 23: | Entrance hole | 270: | Support bracket |
| 24: | Outlet | 280: | Irradiator |
| 30: | Second case | 281: | Printed circuit board |
| 35: | Inner housing | 282: | Sterilizing light source |
| 40: | Filter part | 290: | Battery |
| 70: | Fan module | 300: | Rotation supporter |
| 80: | Fan housing | 310: | Core member |
| 90: | Fan member | 350: | Core supporter |
| 130: | Fan base | 370: | Ball joint |
| 140: | Discharge part | 400: | Sterilizer |
| 150: | First discharge part | 410: | Sterilizing and supporting part |
| 152: | First discharge core | 420: | Support body |
| 154: | First discharge body | 430: | Reflector |
| 156: | Vane | | |
| 160: | Second discharge part | | |
| 161: | Second discharge core | | |
| 162: | Second discharge body | | |
| 163: | Discharging and supporting part | | |
| 200: | Sterilizer | | |
| 210: | Sterilizing and supporting part | | |
| 220: | Support body | | |
| 230: | Reflector | | |
| 232: | First reflecting body | | |
| 234: | Second reflecting body | | |
| 450: | Heat dissipating hole part | | |
| 460: | Blocking body | | |
| 470: | Support part | | |
| 480: | Irradiator | | |
| 500: | Sterilizer | | |
| 510: | Sterilizing and supporting part | | |
| 530: | Reflector | | |
| 550: | Heat dissipating hole part | | |
| 560: | Irradiator | | |
| A: | First area | | |
| B: | Second area | | |
| D: | Predetermined distance | | |
| G1: | First length | | |
| G2: | Second length | | |
| F: | Predetermined angle | | |

## Claims

1. A portable air purifier (1), comprising:
a housing (10) provided with an entrance part (22) forming a passage for suctioning air;
a filter part (40) disposed on an upper side of the entrance part (22) and configured to purify air suctioned into the entrance part (22) and moving upward;
an irradiator (280) disposed on a lower side of the filter part (40), installed at a height where the irradiator (280) overlaps the entrance part (22) and configured to irradiate a light ray for sterilization to the filter part (40); and
a sterilizing and supporting part (210) configured to support the irradiator (280), fixed to the housing (10) and disposed around an outer perimeter of the irradiator (280).

2. The portable air purifier of claim 1, wherein the irradiator (280) comprises:
a printed circuit board (281) supported by the sterilizing and supporting part (210) and disposed on the lower side of the filter part (40); and
a sterilizing light source (282) disposed on the printed circuit board (281) and configured to irradiate a light ray for sterilization to the filter part (40).

3. The portable air purifier of claim 2, wherein centers of the sterilizing light source (282) and the filter part (40) are aligned with each other in an up-down direction.

4. The portable air purifier of claim 2 or 3, wherein the sterilizing and supporting part (210) comprises:
a support body (220) disposed around the outer perimeter of the irradiator (280) and configured to reflect a light ray for sterilization irradiated from the sterilizing light source (282) to the filter part (40).

5. The portable air purifier of claim 4, wherein the support body (220) comprises:
a reflector (230) being concave toward the filter part (40), provided with the sterilizing light source (282) at a center thereof, and configured to reflect light irradiated from the sterilizing light source (282) to the filter part (40); and
an inner supporter (240) extended to a lower side of the reflector (230) and configured to support the printed circuit board (281) of the irradiator (280).

6. The portable air purifier of claim 5, wherein the support body (220) further comprises:
a heat dissipating hole part (250) forming a plurality of holes on the reflector (230) and configured to air-cool the irradiator (280),
wherein optionally the heat dissipating hole part (250) comprises a plurality of long holes having a circular arc shape around the sterilizing light source (282).

7. The portable air purifier of claim 5 or 6, wherein the reflector (230) is disposed on an inner side of the housing (10), facing the entrance part (22).

8. The portable air purifier of any one of claims 5 to 7, wherein the reflector (230) comprises:
a first reflecting body (232) installed along a perimeter of the sterilizing light source (282) and spaced from the sterilizing light source (282); and
a second reflecting body (234) extended from the first reflecting body (232) upward at a slant.

9. The portable air purifier of claim 6, wherein the second reflecting body (234) is formed into a funnel, an upper side of which is open, and has an inner diameter which gradually increases from the first reflecting body (232) toward the filter part (40).

10. The portable air purifier of any one of claims 5 to 9, wherein the inner supporter (240) comprises:
an inner bracket (242) extended to the lower side of the reflector (230); and
a held hook (244) bent from a lower side of the inner bracket (242) inwardly towards the irradiator (280) and holding an edge of the irradiator (280).

11. The portable air purifier of any one of claims 5 to 10, wherein the support body (220) further comprises:
a blocking body (260) extended downward from an outer edge of the reflector (230),
wherein optionally the blocking body (260) is formed into a cylinder, a lower side of which is open, and a center of the blocking body (260) and a center of the entrance part (22) are aligned with each other.

12. The portable air purifier of any one of claims 4 to 11, wherein the sterilizing and supporting part (210) further comprises:
a support bracket (270) extended to a lower side of the support body (220) and fixed to the housing (10).

13. The portable air purifier of any one of claims 1 to 12, wherein:
the housing (10) comprises a first case (20) provided therein with an accommodation space (21), provided with the entrance part (22) for suctioning air on a lateral surface of a lower portion thereof and provided with an outlet (23) for discharging air on an upper side thereof;
the housing (10) comprises a second case (30) connected to the lower portion of the first case (20);
the filter part (40) is installed inside the first case (20) and configured to purify air suctioned into the entrance part (22);
the portable air purifier (1) further comprises a fan module (70) disposed between the filter part (40) and the outlet (23) and configured to rotate a fan (90) to blow air toward the outlet (23); and
the sterilizing and supporting part (210) is fixed to at least one of the first case (20) and the second case (30).

14. The portable air purifier of claim 13, wherein centers of the first case (20), the filter part (40), the fan module (70) and the irradiator (280) are aligned with one another in an up-down direction.

15. The portable air purifier of any one of claims 5 to 14, wherein a reflective coating layer is formed on a surface of the reflector (230).
